# EUROPEAN PATENT APPLICATION

(11) **EP 2 256 652 A2**
(43) Date of publication of application: **01.12.2010**
(21) Application number: 10164152.0
(22) Date of filing: 27.05.2010
(51) Int. Cl.: G06F 19/00

(54) **Radiographic image display apparatus, and its method and computer program product**

(30) Priority: 28.05.2009 JP 2009129014
(71) Applicant: FUJIFILM Corporation, Tokyo 106-8620 (JP)
(72) Inventor: Moriya, Yoshiyuki, Tokyo 107-0052 (JP)
(74) Representative: Klunker . Schmitt-Nilson . Hirsch

(57) **Abstract**

A position of a pathological examination on a radiographic image, such as a CT image and an MRI image, is stored beforehand in association with the radiographic image. When the radiographic image is displayed on a monitor device, a marker indicating the position of the pathological examination, which is stored in association with the human body image, is displayed at a corresponding position on the radiographic image displayed on the monitor device. Further, information (a pathology image and a pathology report) on the pathological examination of a tissue collected from the position of the pathological examination is displayed simultaneously with the radiographic image. Therefore, the position of the pathological examination can be accurately grasped on the radiographic image, and the correspondence relationship between the image at the position (biopsy site) of the pathological examination on the radiographic image, and the information on the pathological examination can be clearly grasped.

## Description

### BACKGROUND OF THE INVENTION

### Field of the Invention

The presently disclosed subject matter relates to a radiographic image display apparatus, and method and computer program product, and more particularly to a technique used to display a medical radiographic image relating to pathological examination.

### Description of the Related Art

Conventionally, when a pathological examination is performed, an anatomical site name is written in a pathological examination report, and a biopsy position is specified on a radiographic image on the basis of the site name.

Further, a medical image diagnostic apparatus is proposed, in which, when a predetermined coordinate position, such as a position of a lung nodule, is set in a three-dimensional medical image, an anatomical site name (lung segment) of the site corresponding to the coordinate position is determined, so that the determined site name is displayed on the medical image in a superimposed manner, or so that a site corresponding to the anatomical site name is highlighted on a schematic diagram (Japanese Patent Application Laid-Open No. 2009-45286).

### SUMMARY OF THE INVENTION

However, an anatomical site name has only information on an area of a segment of an organ, such as a lung. Thus, there is a problem that since the site of a pathological examination cannot be accurately specified on a CT (Computed Tomography) image or an MRI (Magnetic Resonance Imaging) image, the position of the pathological examination cannot be specified in the case where there are different observations in the site area.

Further, the invention described in Japanese Patent Application Laid-Open No. 2009-45286 is configured such that an anatomical site name determined on the basis of a coordinate position is displayed on a medical image in a superimposed manner, or such that a site corresponding to the anatomical site name is highlighted on a schematic diagram. Hence, the invention is not configured to display the position of pathological examination.

The presently disclosed subject matter has been made in view of the above described circumstances. The presently disclosed subject matter aims to provide a radiographic image display apparatus, method and computer program product which enable accurately grasping the position of a pathological examination on a radiographic image, such as a CT image and an MRI image.

To this end, a radiographic image display apparatus according to a first aspect of the presently disclosed subject matter, which makes a display device display a medical radiographic image, includes: a storing device which stores a position of a pathological examination on the radiographic image in association with the radiographic image; a position acquiring device which, when the radiographic image is displayed, acquires, from the storing device, the position of the pathological examination stored in association with the radiographic image; and a display control device which, on the basis of the position of the pathological examination that is stored in the storing device and acquired by the position acquiring device, makes the display device visibly display the position of the pathological examination on the radiographic image displayed by the display device.

That is, the radiographic image display apparatus is configured such that, when a radiographic image, such as a CT image and an MRI image, is displayed, on the basis of a position of a pathological examination, the position stored in association with the radiographic image, the position of the pathological examination is visibly displayed on the radiographic image, thereby enabling to accurately grasp the position of the pathological examination on the radiographic image. Note that as a method for visibly displaying a position of a pathological examination, it is conceivable to display a marker at the position of the pathological examination on the radiographic image, or to change the density or color of the pathological range corresponding to the position of the pathological examination.

According to a second aspect of the presently disclosed subject matter, the radiographic image display apparatus according to the first aspect, further includes: an image acquiring device which acquires a radiographic image including a site of a pathological examination; a position specifying device which makes the display device display the acquired radiographic image and which specifies a position of a pathological examination on the displayed radiographic image; and a registering device which makes the storing device store, in association with the radiographic image, the position of the pathological examination specified by the position specifying device.

According to a third aspect of the presently disclosed subject matter, in the radiographic image display apparatus according to the second aspect, the position specifying device includes a pointing device which moves a pointer to a desired position on the radiographic image displayed by the display device, and the registering device makes the storing device store the position on the radiographic image specified by the pointing device, as the position of the pathological examination in association with the radiographic image.

According to according to a fourth aspect of the presently disclosed subject matter, an image acquiring device which acquires radiographic images before and after the pathological examination, each of the images includes a site of a pathological examination; a position specifying device which makes the display device display the acquired radiographic image after the pathological examination and which specifies the position of the pathological examination on the displayed radiographic image; a position converting device which converts the position of the pathological examination specified on the radiographic image after the pathological examination to the position of the pathological examination on the radiographic image before the pathological examination; and a registering device which makes the storing device store, in association with the radiographic image after the pathological examination, the position of the pathological examination specified by the position specifying device, and the display control device makes the display device visibly display, on the radiographic image before the pathological examination displayed by the display device, the position of the pathological examination converted by the position converting device.

According to a fifth aspect of the presently disclosed subject matter, the radiographic image display apparatus according to the first aspect further includes: an image acquiring device which acquires radiographic images before and after the pathological examination, each of the images includes a site of a pathological examination; a position specifying device which makes the display device display the acquired radiographic image after the pathological examination and which specifies the position of the pathological examination on the displayed radiographic image; a position converting device which converts the position of the pathological examination specified on the radiographic image after the pathological examination to the position of the pathological examination on the radiographic image before the pathological examination; and a registering device which makes the storing device store, in association with the radiographic image before the pathological examination, the position of the pathological examination, which position is converted by the position converting device.

That is, the invention according to one of the fourth and fifth aspects, is configured such that a radiographic image after a pathological examination (after a biopsy, and the like, is actually performed) is used as an image for specifying the position of the pathological examination, and that the position of the pathological examination is visibly displayed on the radiographic image before the pathological examination (before the biopsy, and the like, is performed) on the basis of the position of the pathological examination, which position is specified on the radiographic image after the pathological examination. This is because an image corresponding to a pathological examination result is not an image as a result of the pathological examination (after biopsy) but an image before the biopsy, and thus it is desirable to display the position of the pathological examination on the image before the biopsy.

Note that the position converting device performs the alignment of two radiographic images before and after a pathological examination whereby a position of the pathological examination specified on the radiographic image after the pathological examination can be converted to a position of the pathological examination on the radiographic image before the pathological examination. Further, the invention according to the fourth aspect is configured to store a position of a pathological examination on a radiographic image before the pathological examination. On the other hand, the invention according to the fifth aspect is configured to store a position of a pathological examination, which position is converted by the position converting device to a position of the pathological examination on a radiographic image after the pathological examination.

According to a sixth aspect of the presently disclosed subject matter, the radiographic image display apparatus according to the one of the fourth and fifth aspects, includes, instead of the position specifying device, a position detecting device which detects, by image processing, the position of the pathological examination from the acquired radiographic image after the pathological examination.

For example, when a biopsy of a lesion area is performed by partially excising the lesion area in a VATS (Video-Assisted Thoracic Surgery), or the like, there is a case where, because a metal is left in the lesion area, the position at which the VATS is performed can be detected by applying image processing to the image after the VATS.

According to a seventh aspect of the presently disclosed subject matter, in the radiographic image display apparatus according to the second aspect, the position specifying device includes a device which specifies the position of the pathological examination on the basis of an input of one of a name and a symbol which indicate an anatomical site. That is, the position specifying device is not limited to the device which specifies a position (coordinates) on a radiographic image, but may include a device which specifies (uniquely specifies) a position of a pathological examination on the basis of an input of a name or a symbol which indicates an anatomical site. In this case, it is necessary to specify an anatomical site on the basis of the radiographic image and to obtain a correspondence relationship between the specified anatomical site and the name or symbol which indicates the anatomical site.

According to an eighth aspect of the presently disclosed subject matter, in the radiographic image display apparatus which is applied to a pathological examination performed in such a manner that the distal end of a bronchoscope is inserted to reach a desired position of the bronchus, a sterile physiological saline solution is injected into a lung segment distal to the insertion position the injected solution is sucked back, and cells, protein, and microorganisms in the sucked back saline solution are examined, the display control device includes a recognizing device which, when the distal end position of the bronchoscope inserted into the bronchus, or one of a name and a symbol that indicate the anatomical site corresponding to the distal end position of the bronchoscope is inputted by the position specifying device, recognizes, from the radiographic image, a dominated area of the bronchus which is distal to the distal end position of the bronchoscope, and the display control device makes the display device visually display the recognized dominated area of the bronchus in a manner that the recognized dominated area of the bronchus can be discriminated from the other regions on the radiographic image displayed by the display device.

According to a ninth aspect of the presently disclosed subject matter, the radiographic image display apparatus according to one of the first to eighth aspects, further includes: a pathology database which manages information on a pathological examination of a tissue collected from a position of the pathological examination, and the display control device makes the display device visibly display the position of the pathological examination on the radiographic image displayed by the display device and reads the information of the corresponding pathological examination from the pathology database to make the display device display the read information. This makes it possible to clearly grasp the correspondence relationship between the image at the position of the pathological examination (biopsy site) on the radiographic image and the information on the pathological examination. Note that the information on the pathological examination is information including at least one of a pathology image, such as a microphotograph of the collected tissue, and a pathology report.

According to a tenth aspect of the presently disclosed subject matter, in the radiographic image display apparatus according to the ninth aspect, the pathology database also serves as a storing device which stores, in association with the radiographic image, the position of the pathological examination on the radiographic image.

According to an eleventh aspect of the presently disclosed subject matter, the radiographic image display apparatus according to one of the first to tenth aspects, further includes: a pathology database which manages information on a pathological examination of a tissue collected from a position of the pathological examination; and
a selecting device which selects information on a desired pathological examination from the information on the pathological examination registered in the pathology database, and when the information on the desired pathological examination is selected by the selecting device, the display control device reads out corresponding information on the pathological examination from the pathology database, and makes the display device display the read information on the pathological examination and the radiographic image corresponding to the information on the pathological examination. The invention according to the eleventh aspect is configured such that, when information on a desired pathological examination is displayed, the radiographic image which enables the position of the pathological examination to be visually recognized is simultaneously displayed.

According to a twelfth aspect of the presently disclosed subject matter, in the radiographic image display apparatus according to the eleventh aspect, the selecting device includes at least one of: a first selecting device which retrieves information on a desired pathological examination from the pathology database by an input of retrieval information; and a second selecting device which includes a device which makes the display device display a list of information on the pathological examination registered in the pathology database, and a device which selects information on a desired pathological examination from the list.

According to thirteenth aspect of the presently disclosed subject matter, the radiographic image display apparatus according to one of the first to twelfth aspects, further includes an image database which manages medical radiographic images, and the position of the pathological examination on the radiographic image is recorded in a tag of an image file of the radiographic image For example, it is conceivable to record the position of the pathological examination in a private tag, and the like, of an image file based on the DICOM (Digital Image and Communication in Medicine) standard.

According to a fourteenth aspect of the presently disclosed subject matter, the radiographic image display apparatus according to one of the first to thirteenth aspects, further includes a mode selecting device which selects one of a display mode and a non-display mode of a position of a pathological examination, and the display control device makes the position of the pathological examination visibly displayed on the radiographic image only when the display mode is selected by the mode selecting device.

When a position of a pathological examination is displayed by a marker, and the like, on a radiographic image, the position of the pathological examination can be accurately grasped. However, it is possible to consider a case where the display of the marker, and the like, interferes with the image diagnosis. To cope with this case, it is configured such that, when the non-display mode is selected, only the original radiographic image is displayed without the position of the pathological examination being displayed.

A fifteen aspect of the presently disclosed subject matter provides a radiographic image display method for making a display device display a medical radiographic image, comprising the steps of: making a storing device store a position of a pathological examination on the radiographic image in association with the radiographic image; acquiring, when the radiographic image is displayed, from the storing device, the position of the pathological examination stored in association with the radiographic image; and making the display device visibly display the position of the pathological examination on the radiographic image displayed by the display device, on the basis of the acquired position of the pathological examination.

According to a sixteenth aspect, the radiographic image display method according to the fifteenth aspect, further includes the steps of: acquiring a radiographic image including a pathological examination site; making the display device display the acquired radiographic image and specifying a position of the pathological examination on the displayed radiographic image; and making the storing device store the specified position of the pathological examination in association with the radiographic image.

A seventeenth aspect of the presently disclosed subject matter provides a computer program product stored on a computer readable medium for making a computer perform radiographic image display control, the product includes computer-executable instructions of: making a storing device store a position of a pathological examination on the radiographic image in association with the radiographic image; acquiring, when the radiographic image is displayed, from the storing device, the position of the pathological examination stored in association with the radiographic image; and making the display device visibly display the position of the pathological examination on the radiographic image displayed the display device, on the basis of the acquired position of the pathological examination.

According to an eighteenth aspect of the presently disclosed subject matter, the computer program product according to the seventeenth aspect, further includes computer-executable instructions of: acquiring a radiographic image including a pathological examination site; making the display device display the acquired radiographic image and receiving specification of a position of the pathological examination on the displayed radiographic image; and making the storing device store the specified position of the pathological examination in association with the radiographic image.

According to the presently disclosed subject matter, it becomes possible that a position of a pathological examination is stored in association with a radiographic image, such as a CT image and an MRI image, and when the radiographic image is displayed, the position of the pathological examination is visibly displayed on the radiographic image on the basis of the position of the pathological examination stored in association with the radiographic image. Thus, the position of the pathological examination can be accurately grasped on the radiographic image.

Further, it becomes possible that information (a pathology image and a pathology report) on the pathological examination of a tissue collected from the position of the pathological examination is displayed simultaneously with the radiographic image. This enables to clearly grasp a correspondence relationship between the image at the position of the pathological examination (biopsy site) on the radiographic image and the information on the pathological examination.

### BRIEF DESCRIPTION OF THE DRAWINGS

Fig. 1 is a diagram showing a system configuration including a radiographic image display apparatus according to the presently disclosed subject matter;
Fig. 2 is a flow chart showing a processing procedure for registering a position, and the like, of a pathological examination on a radiographic image in a pathology DB by the radiographic image display apparatus according to a first embodiment;
Fig. 3 is a diagram showing an example of an image DB;
Fig. 4 is a diagram showing an example of the pathology DB which manages the information on pathological examination and the position of pathological examination;
Fig. 5 is a flow chart of the first embodiment showing a processing procedure for displaying the information registered in the image DB and the pathology DB by the radiographic image display apparatus;
Fig. 6 is a diagram showing a display image which is displayed by a monitor device and which includes a human body image (CT image), a pathological examination result, and a pathology image;
Fig. 7 is a flow chart showing a processing procedure for registering a position, and the like, of a pathological examination on a radiographic image in the pathology DB by the radiographic image display apparatus according to a second embodiment;
Fig. 8 is a flow chart showing a processing procedure for displaying the information registered in the image DB and the pathology DB by the radiographic image display apparatus according to the second embodiment;
Fig. 9 is a flow chart showing a processing procedure for registering a position, and the like, of a pathological examination on a radiographic image in the pathology DB by the radiographic image display apparatus according to a third embodiment;
Fig. 10 is a flow chart showing a processing procedure for displaying the information registered in the image DB and the pathology DB by the radiographic image display apparatus according to the third embodiment; and
Fig. 11 is a diagram showing a display image in which a dominated area of the bronchus corresponding to a position (area) of a pathological examination is displayed so as to be distinguishable from the other regions.

### DETAILED DESCRIPTION OF THE PREFERRED EMBODIMENTS

In the following, preferred embodiments of a radiographic image display apparatus according to the presently disclosed subject matter, and a method and program used in the radiographic image display apparatus will be described with reference to the accompanying drawings.

### [Apparatus configuration]

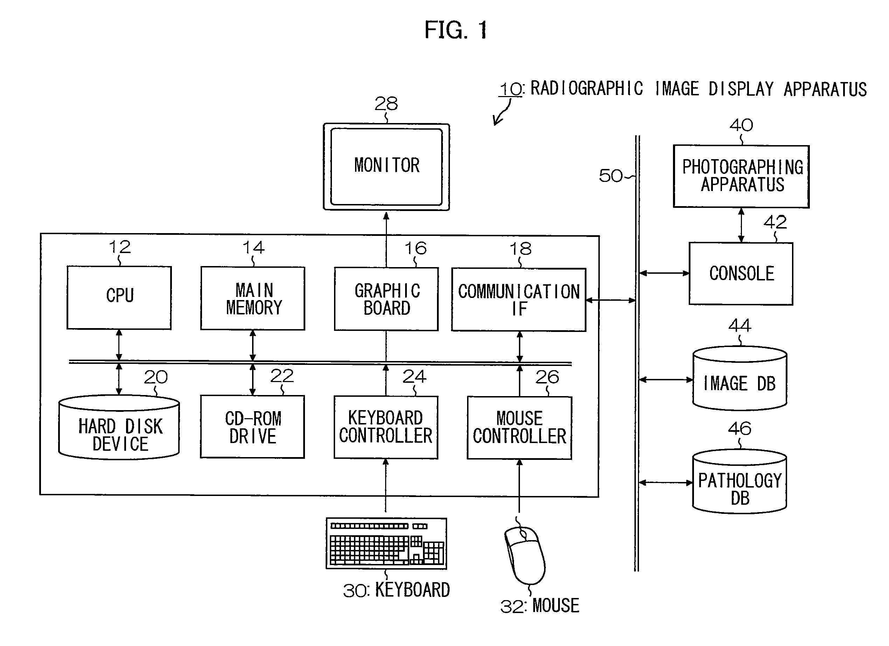

Fig. 1 is a diagram showing a system configuration including a radiographic image display apparatus according to the presently disclosed subject matter.

The system includes a radiographic image display apparatus 10 according to the presently disclosed subject matter, a photographing apparatus 40 installed at a medical facility, and the like, a console 42 which is used to perform an operation, and the like, of the photographing apparatus 40, an image database (image DB) 44 which stores a medical radiographic image, and the like, photographed by using the photographing apparatus 40, and a pathology database (pathology DB) 46 which manages information on a pathological examination, obtained as a result of the pathological examination of a tissue, cells, and the like, collected from a position of the pathological examination.

The radiographic image display apparatus 10 can be configured using a computer, such as a workstation, and is mainly configured by: a central processing unit (CPU) 12 which mainly controls the operation of respective components; a main memory 14 in which the control program of the apparatus is stored and which serves as a work area at the time when the program is executed; a graphic board 16 which controls the display of a monitor device 28, such as a liquid crystal display and a CRT (Cathode ray Tube) display; a communication interface (communication I/F) 18 which is connected to a network 50 of the medical facility; a hard disk device 20 in which a radiographic image display program according to the presently disclosed subject matter, various kinds of application software including an image-analysis program, an operation system, and the like, are stored; a CD-ROM drive 22 which reads information from a CD-ROM on which various kinds of information, such as a program and a human body image, are recorded, and which makes the read information stored in the hard disk device 20; a keyboard controller 24 which detects a key operation of a keyboard 30 and outputs, to the CPU 12, the detected key operation as an instruction input; and a mouth controller 26 which detects the state of a mouse 32 as a position specifying device, and outputs, to the CPU 12, signals relating to a position of a mouse pointer on the monitor device 28, the state of the mouse 32, and the like.

Note that an X-ray CT apparatus, an MRI (Magnetic Resonance Imaging) apparatus, and the like, can be considered as the photographing apparatus 40, but the presently disclosed subject matter is not limited to these. The presently disclosed subject matter may be applied to any apparatus as long as the apparatus photographs a medical radiographic image. Further, it is assumed that the radioactive ray in the presently disclosed subject matter means radioactive rays in a broad sense, which include particle rays, such as electron beams, and electromagnetic waves, in addition to general radioactive rays, such as X-rays, α rays, β rays, γ rays, and ultraviolet rays.

### <First embodiment>

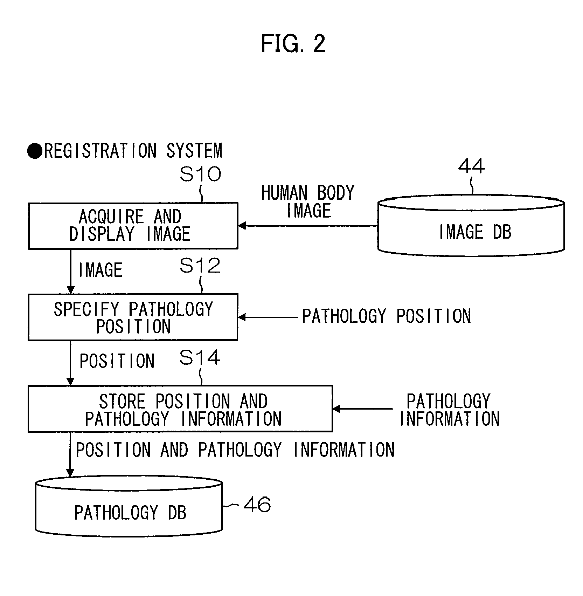

In the following, a first embodiment according to the presently disclosed subject matter will be described with reference to Fig. 2 to Fig. 6.

### [Registration system]

Fig. 2 is a flow chart showing a processing procedure for registering a position, and the like, of a pathological examination on a radiographic image in the pathology DB 46 by the radiographic image display apparatus 10 according to a first embodiment.

The CPU 12 executes a radiographic image display program according to an input operation by the keyboard 30 and the mouse 32, to function as an image acquiring device which acquires a desired radiographic image (hereinafter referred to as "human body image") from the image DB 44 via the network 50 and the communication I/F 18, and makes the monitor device 28 display the acquired human body image via the graphic board 16 (step S10). Note that a file (DICOM file) based on the DICOM (Digital Imaging and Communications in Medicine) standard can be considered as a format of a file in which the human body image is stored.

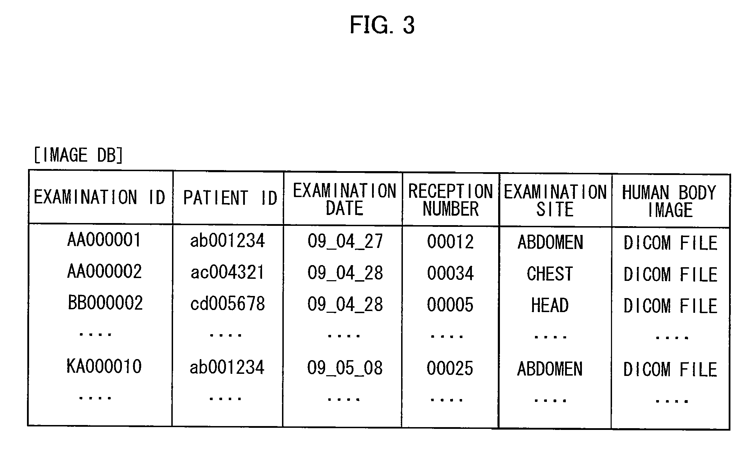

Fig. 3 is a diagram showing an example of the image DB 44.

In the image DB 44, human body images are managed in association with the examination identification information (examination ID), the patient ID, the examination date, the reception number, and the examination site. Note that in the present embodiment, the examination ID serves as a main key which uniquely identifies the human body image. Further, the information about the patient and the human body image is stored in the header of the DICOM (Digital Imaging and Communications in Medicine) file, and hence the information, such as the patient ID, can be acquired from the header of the DICOM file.

When an examination ID is inputted by the keyboard 30 at the time of selection of a human body image, the CPU 12 acquires, from the image DB 44, the human body image (for example, a human body image including an examination site of a patient subjected to a pathological examination) uniquely specified by the examination ID, and makes the monitor device 28 display the acquired human body image. Note that it may also be configured such that a list of information registered in the image DB 44 is displayed by the monitor device 28 and such that a desired human body image is acquired by selecting the desired human body image by using the mouse 32.

When the human body image acquired as described above is a CT image and for example, is a series of slice tomograms (three-dimensional volume image) laminated for each slice position in the body axis direction, it is possible that the slice tomograms are stacked and displayed (displayed in a stacked manner) by the monitor device 28, and that a slice tomogram at a desired slice position including an examination site is displayed in accordance with selection indicated by rotation the wheel of the mouse 32.

Then, the position of the pathological examination on the human body image displayed by the monitor device 28 is specified (step S12). The specification of the position of the pathological examination is performed in such a manner that the position of the pathological examination on the human body image is visually checked and the mouse cursor is moved to the position.

When the position of the pathological examination is specified by the mouse cursor on the human body image displayed by the monitor device 28 and is instructed to be registered, the CPU 12 registers the position of the pathological examination into the pathology DB 46 in association with the displayed human body image (step S 14).

Further, in step S14, when the information on the pathological examination, which is information created on the basis of a tissue collected from the position of the pathological examination, is inputted, the CPU 12 registers the information on the pathological examination in the pathology DB 46. The information on the pathological examination includes a pathology image, such as a microphotograph of the collected tissue, and a pathology report indicating the result of the pathological examination.

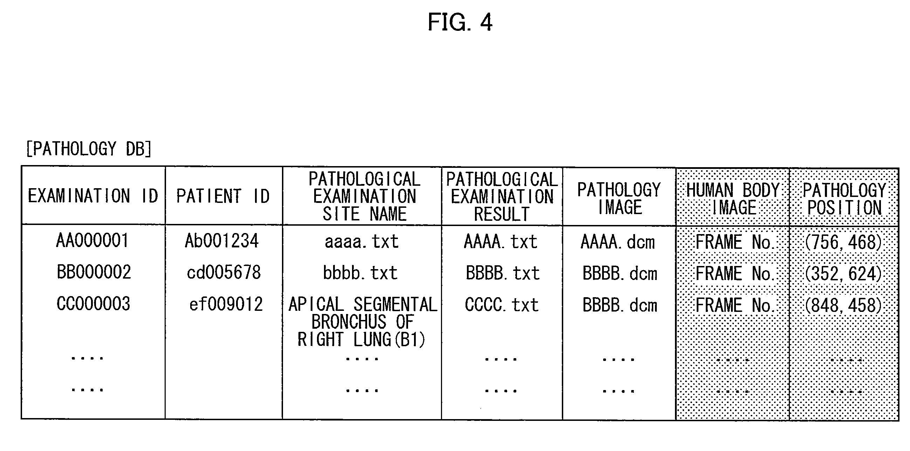

Fig. 4 is a diagram showing an example of the pathology DB 46 which manages the position of pathological examination and the information on pathological examination that are registered as described above.

In the pathology DB 46, information on a pathological examination which includes a pathological examination result that is text information, such as a pathology report; and a pathology image, and a position of the pathological examination (a frame number of a human body image and a coordinate position on the human body image) are managed in association with an examination ID, a patient ID, and a site name of the pathological examination.

In the present embodiment, the examination ID serves as a main key used to uniquely specify a pathological examination, and further the same examination ID as the examination ID of the image DB 44 shown in Fig. 3 is used. This enables to retrieve a human body image corresponding to a pathological examination from the image DB 44 on the basis of an examination ID managed in the pathology DB 46, and to retrieve information on a pathological examination corresponding to a human body image from the pathology DB 46 on the basis of an examination ID managed in the image DB 44.

The position of the pathological examination and the information on the pathological examination are registered in the pathology DB 46 as described above.

Note that in the present embodiment, a human body image and a position of a pathological examination (information on the pathological examination) are associated with each other by an examination ID. However, in the case where a series of slice tomograms (a plurality of human body images) are associated with one examination ID as described above, a human body image including an actual pathological examination site can be specified by further specifying the frame number (see Fig. 4) of one of the plurality of human body images.

Further, the sequence for registering the position of the pathological examination and the information on the pathological examination into the pathology DB 46 is not limited to the sequence of the flow chart shown in Fig. 2, but the registration can be arbitrarily performed. For example, the registration may be performed such that: a position of a pathological examination is registered in the pathology DB 46 at the time of a biopsy; and then the pathological examination of a collected tissue is performed; and the result of the pathological examination and the pathology image obtained by microscopically photographing the tissue are registered in the pathology DB 46. Alternatively, the registration may be performed in a manner that, after the result of the pathological examination is registered in the pathology DB 46, the position of the pathological examination is additionally registered in the pathology DB 46.

### [Display system]

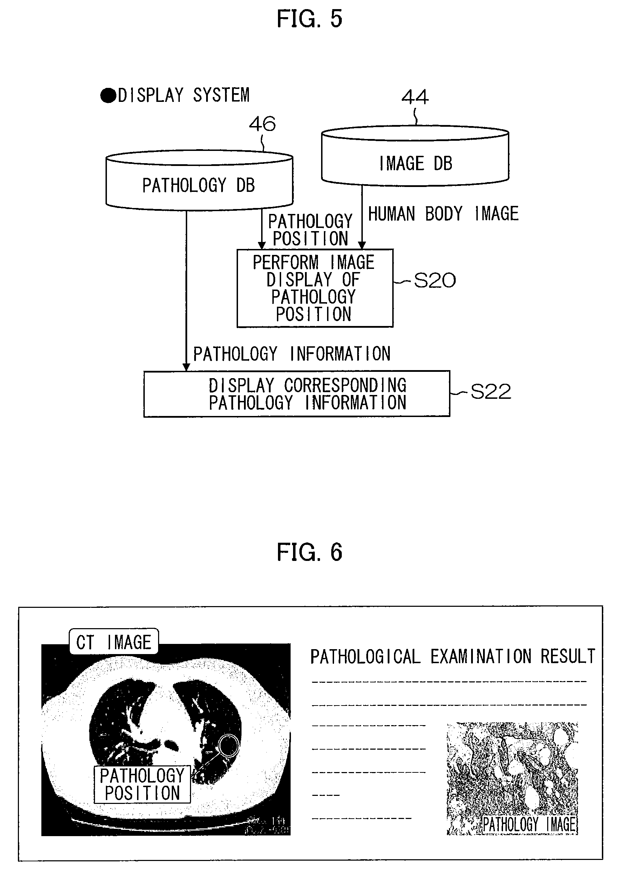

Fig. 5 is a flow chart of the first embodiment showing a processing procedure in which the information registered in the image DB 44 and the pathology DB 46 is displayed by the radiographic image display apparatus 10.

The CPU 12 selects a desired human body image from the image DB 44 according to an input operation performed by the keyboard 30 or the mouse 32 similarly to the case of registration as described above, and makes the monitor device 28 display the selected human body image (step S20).

Then, in the case where the information on the pathological examination and the position of the pathological examination, which correspond to the selected human body image, are registered in the pathology DB 46, the CPU 12 reads out the information and the position from the pathology DB 46. On the basis of the read position of the pathological examination, the CPU 12 makes the monitor device 28 visibly display the position of the pathological examination on the human body image displayed by the monitor device 28 (step S20). Further, the CPU 12 makes the monitor device 28 display the read information on the pathological examination (step S22).

Fig. 6 is a diagram showing a display image of a human body image (CT image), a pathological examination result, and a pathology image which are displayed by the monitor device 28.

As shown in Fig. 6, in the case where a plurality of slice tomograms, such as CT images, are displayed as human body images, when a position of a pathological examination exists on one of the slice tomograms which are stacked and displayed (when a slice tomogram having a frame number registered in the pathology DB 46 shown in Fig. 4 is displayed), on the basis of the coordinate values of the pathology position registered in the pathology DB 46, the CPU 12 makes a marker (circular marker having a predetermined size and centering at the coordinate values) displayed in a superimposed manner at the position of the pathological examination on the human body image, and makes the result of the pathological examination and the pathology image displayed side by side together with the human body image.

The biopsy site can be accurately grasped on the human body image by the marker as described above. Particularly, when there are different observations in the same site area, the position of the biopsy site can be specified in the site area by the marker. Further, it is configured such that the information (the pathology image and the pathology report) on the pathological examination of the tissue collected from the biopsy site can be displayed simultaneously with the human body image. Thus, the correspondence relationship between the image at the biopsy site on the human body image and the information on the pathological examination can be clearly grasped, so that the image reading technique can be improved.

### <Second embodiment>

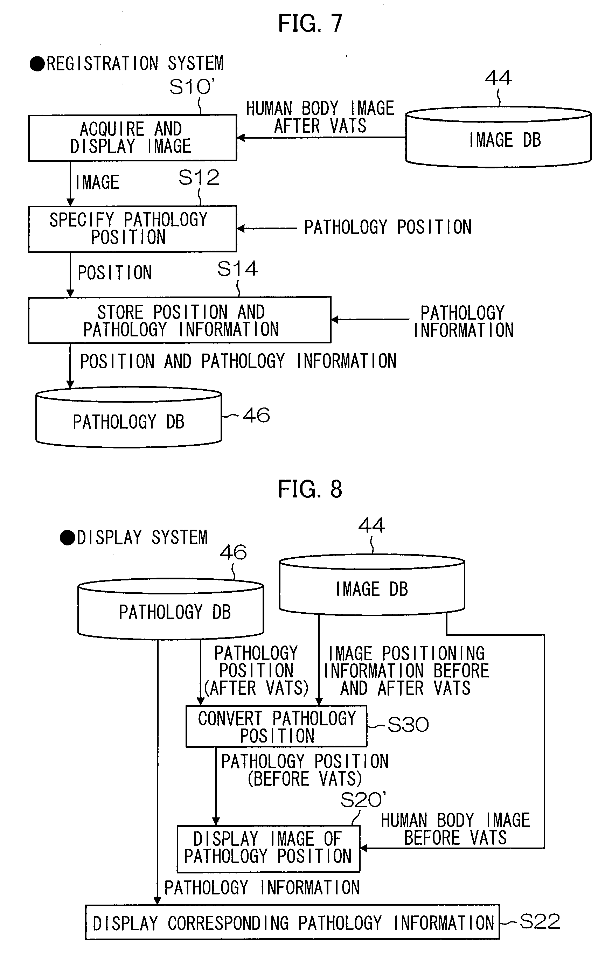

Next, a second embodiment according to the presently disclosed subject matter will be described with reference to Fig. 7 and Fig. 8.

### [Registration system]

Fig. 7 is a flow chart of showing a processing procedure for registering a position, and the like, of a pathological examination on a radiographic image in the pathology DB 46 by the radiographic image display apparatus 10 according to a second embodiment. Note that the steps of the flow chart of the second embodiment that are common to the steps of the flow chart of the first embodiment shown in Fig. 2 are designated by the same step numbers, and the detailed description thereof is omitted.

The first embodiment is configured such that a marker indicating a position of a pathological examination (biopsy site) at which position the biopsy was performed is displayed on a human body image including the position of the pathological examination. However, the second embodiment is different from the first embodiment in that the position of the pathological examination is specified on the human body image after the pathological examination and in that the marker indicating the position of the pathological examination is displayed on the human body image before the pathological examination.

When a biopsy of a lesion area is performed by partially excising the lesion area in a VATS (Video-Assisted Thoracic Surgery), or the like, there is a case where, because a metal is left in the lesion area, or other reasons, the position at which the VATS is performed can be detected by applying image processing to the image after the VATS.

In Fig. 7, the CPU 12 acquires a human body image after a VATS according to an input operation performed by the keyboard 30 or the mouse 32, and makes the monitor device 28 display the acquired human body image (step S10).

Note that the item of VATS information indicating whether an image is taken before or after the VATS is not included in the image DB 44 shown in Fig. 3. However, only the human body image after the VATS (image taken after the VATS) can be extracted from the image DB 44 when the VATS information is managed in the image DB 44. Further, in the case where there are human body images which are specified by the same patient ID and the same examination site, and which have different examination dates, the human body image after the VATS can be extracted by selecting the human body image having the examination date after the date when the VATS was performed.

Subsequently, similarly to the first embodiment, a position of the pathological examination is specified on the human body image displayed by the monitor device 28 (step S12). Note that the method for specifying the position of the pathological examination is not limited to the method in which the position of the pathological examination is manually specified by visually checking the biopsy site on the human body image. However, a position can be automatically detected by applying image processing to the image of a metal left in the biopsy site by the VATS (this image has a large CT value because the X-rays hardly transmits through the metal) or the images before and after the VATS, and by specifying the detected position as the position of the pathological examination.

The position of the pathological examination specified as described above and the information on the pathological examination are registered in the pathology DB 46 in association with the human body image after the VATS similarly to the first embodiment (step S14).

### [Display system]

Fig. 8 is a flow chart showing a processing procedure for displaying the information registered in the image DB 44 and the pathology DB 46 by the radiographic image display apparatus 10 according to the second embodiment.

The CPU 12 selects a desired human body image before the VATS from the image DB 44 according to an input operation performed by the keyboard 30 or the mouse 32, and makes the monitor device 28 display the selected human body image (step S20').

On the other hand, in the case where the information on the pathological examination after the VATS and the position of the pathological examination, which correspond to the selected human body image before the VATS, are registered in the pathology DB 46, the CPU 12 reads out these kinds of information from the pathology DB 46, and makes the monitor device 28 display these kinds of information (step S22).

Further, the CPU 12 converts the read position of the pathological examination (the position on the human body image after the VATS) into a corresponding position of the pathological examination on the human body image before the VATS (step S30). Then, on the basis of the converted position of the pathological examination, the CPU 12 makes a marker displayed in a superimposed manner at the position to be subjected to the pathological examination on the human body image before the VATS, which is displayed by the monitor device 28 (step S20').

Here, in order to convert the position of the pathological examination on the human body image after the VATS into the position of the pathological examination on the human body image before the VATS in step S30, the CPU 12 creates the image positioning information before and after the VATS on the basis of the two human body images before and after the VATS registered in the image DB 44. Or, the CPU 12 reads out the image positioning information before and after the VATS registered in the image DB 44, and, on the basis of the image positioning information, converts the position of the pathological examination on the human body image after the VATS into the position of the pathological examination on the human body image before the VATS.

The two human body images before and after the VATS do not usually coincide with each other because of the difference in the photographing dates and times, and the difference in the conditions, such as the attitude of the subject at the time of photographing. Thus, anatomical characteristic points (corresponding points) of the two human body images are detected, and the image positioning information (conversion parameters) used for geometrically converting the human body image after the VATS is calculated so that the corresponding points of the human body image after the VATS coincide with the corresponding points of the human body image before the VATS.

Note that the projective transformation using projective transformation parameters, the affine transformation using affine transformation parameters, the Helmert transformation using Helmert transformation parameters, and the like, can be considered as the geometric conversion. Further, in the present embodiment, the coordinate values of the position of the pathological examination on the human body image before the VATS are obtained by geometrically converting the coordinate values of the position of the pathological examination which is specified on the human body image after the VATS, not by geometrically converting the human body image after the VATS, using the image positioning information calculated as describe above. Further, the method for calculating the image positioning information of the human body images before and after the VATS is not limited to the above described example, and a known method, such as the method described in Japanese Patent Application Laid-Open No. 2007-68554, can be used.

According to the second embodiment of the presently disclosed subject matter, a position of a pathological examination can be displayed on a human body image before a biopsy (VATS). An image which has a feature of a lesion area and which corresponds to a result of a pathological examination is not an image after a VATS but is an image before the VATS. Thus, it is desirable to display the position of the pathological examination on the human body image before the VATS, as in the case of the second embodiment according to the presently disclosed subject matter.

Note that the calculation of the image positioning information of the human body images before and after the VATS can be performed at the time when the position of the pathological examination is specified on the human body image after the VATS, and the image positioning information is managed in the image DB 44 in association with the human body image after the VATS. Also, the image positioning information of the human body images before and after the VATS can be calculated on the basis of the human body images before and after the VATS each time the position of the pathological examination is displayed.

Further, the conversion processing of the position of the pathological examination on the human body image before the VATS can be performed based on the above described image positioning information of the human body image before the position of the pathological examination is displayed, and the converted position of the pathological examination can be managed in the image DB 44, and the like, in association with the human body image before the VATS. In this case, when the human body image before the VATS is displayed, a marker can be displayed at the position of the pathological examination without the position of the pathological examination being acquired from the pathology DB 46.

### <Third embodiment>

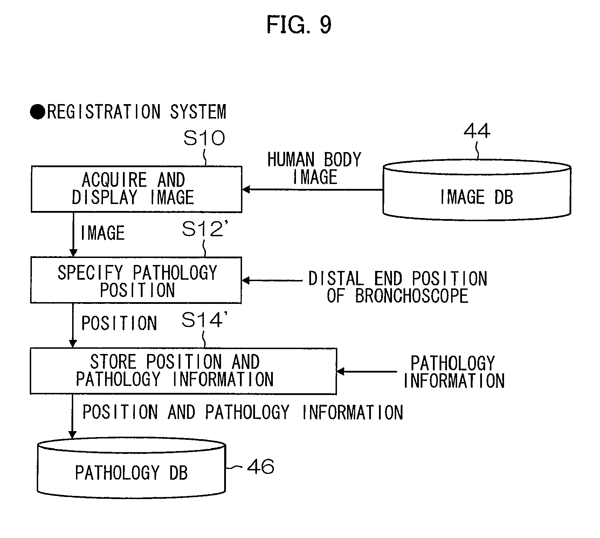

Next, a third embodiment according to the presently disclosed subject matter will be described with reference to Fig. 9 to Fig. 11.

### [Registration system]

Fig. 9 is a flow chart showing a processing procedure for registering a position, and the like, of a pathological examination on a radiographic image in the pathology DB 46 by the radiographic image display apparatus 10 according to a third embodiment. Note that the steps of the flow chart of the third embodiment that are common to the steps of the flow chart of the first embodiment shown in Fig. 2 are designated by the same step numbers, and the detailed description thereof is omitted.

The third embodiment corresponds, for example, to the case where when bronchial alveolar lavage (BAL) is performed, and the method for specifying the position of the pathological examination (biopsy site) is different from those in the first and second embodiments.

The BAL means a method in which the distal end of a bronchoscope is inserted to reach the third or fourth branch of the bronchus, and with the distal end inserted, a sterile physiological saline solution is injected and then the injected solution is sucked back, and cells, protein, and microorganisms in the sucked back saline solution are examined. Therefore, the lung segment distal (on the front side) from the distal end of the inserted bronchoscope is set as the position (range) of the pathological examination.

In Fig. 9, the CPU 12 acquires a desired human body image according to an input operation performed by the keyboard 30 or the mouse 32, to make the monitor device 28 display the acquired human body image (step S10). In the third embodiment i a human body image including a lung field is selected and the human body image is displayed.

Subsequently, as a position of a pathological examination on the human body image displayed by the monitor device 28, the distal end position of the bronchoscope is specified by performing an input operation using the keyboard 30, the mouse 32, or the like (step S 12').

The distal end position of the bronchoscope can be specified by inputting, for example, an anatomical name or a symbol of a bronchial branch by using the keyboard 30. Note that anatomical names and symbols of the bronchial branches include a total of twenty sets of anatomical name and symbol: apical segmental bronchus (B1), posterior segmental bronchus (B2), anterior segmental bronchus (B3), lateral segmental bronchus (B4), medial segmental bronchus (B5), superior segmental bronchus (B6), medial basal segmental bronchus (B7), anterior basal segmental bronchus (B8), lateral basal segmental bronchus (B9), and posterior basal segmental bronchus (B 10) of the right lung, and apical segmental bronchus (B1), posterior segmental bronchus (B2), anterior segmental bronchus (B3), superior lingular bronchus (B4), inferior lingular bronchus (B5), superior segmental bronchus (B6), medial basal segmental bronchus (B7), anterior basal segmental bronchus (B8), lateral basal segmental bronchus (B9), and posterior basal segmental bronchus (B 10) of the left lung.

The position of the pathological examination (the distal end position of the bronchoscope) specified as described above, and the information on the pathological examination performed by BAL in the lung segment distal to the distal end position of the bronchoscope are registered in the pathology DB 46 in association with the human body image (step S14').

The above described position of the pathological examination (the distal end position of the bronchoscope) can be registered in the column of the pathological examination site name in the pathology DB 46 (see Fig. 4).

### [Display system]

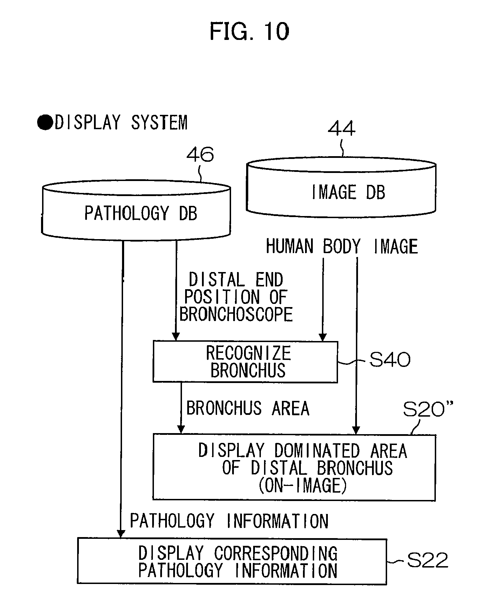

Fig. 10 is a flow chart showing a processing procedure for displaying the information registered in the image DB 44 and the pathology DB 46 the radiographic image display apparatus 10 according to the third embodiment.

The CPU 12 selects a human body image of a patient subjected to BAL from the image DB 44 according to an input operation performed by the keyboard 30 or the mouse 32, so as to make the monitor device 28 display the acquired human body image (step S20").

On the other hand, when the information on the pathological examination corresponding to the human body image of the patient subjected to BAL and the position of the pathological examination (the distal end position of the bronchoscope) have already been registered in the pathology DB 46, the CPU 12 reads out these kinds of information from the pathology DB 46, so as to make the monitor device 28 display the read information on the pathological examination (step S22).

Further, the CPU 12 image-recognizes the corresponding bronchial branch on the human body image on the basis of the read position of the pathological examination (the distal end position of the bronchoscope), and recognizes a dominated area of the bronchus which is distal to the recognized bronchial branch (step S40). Then, the CPU 12 makes the recognized dominated area of the bronchus displayed in a manner that the recognized dominated area can be distinguished from the other regions on the human body image displayed by the monitor device 28 (step S20").

The recognition of the dominated area of the bronchus is described in the following references.

### (Reference 1)

Technical Report of IEICE (the Institute of Electronics, Information and Communication Engineers) MI2002-79 (2002-11), "Primary Investigation on Classification of Lung Field Based on Bronchus Using Chest Multi-slice CT Images"

"The area occupied by the bronchial branches" in Reference 1 described above corresponds to the dominated area.

Further, in the case where the position (area) of the pathological examination on the human body image is specified by a name, and the like, at the distal end position of the bronchoscope, it is necessary to recognize a bronchial branch on the basis of the image as described in the following reference 2, and to obtain a correspondence relationship between the name of the bronchial branch and the position on the image.

### (Reference 2)

Technical Report of IEICE (the Institute of Electronics, Information and Communication Engineers) MI2004-110 (2005-1), Ema Shinya, "Improved model selection method for automated anatomical labeling of bronchial branches"

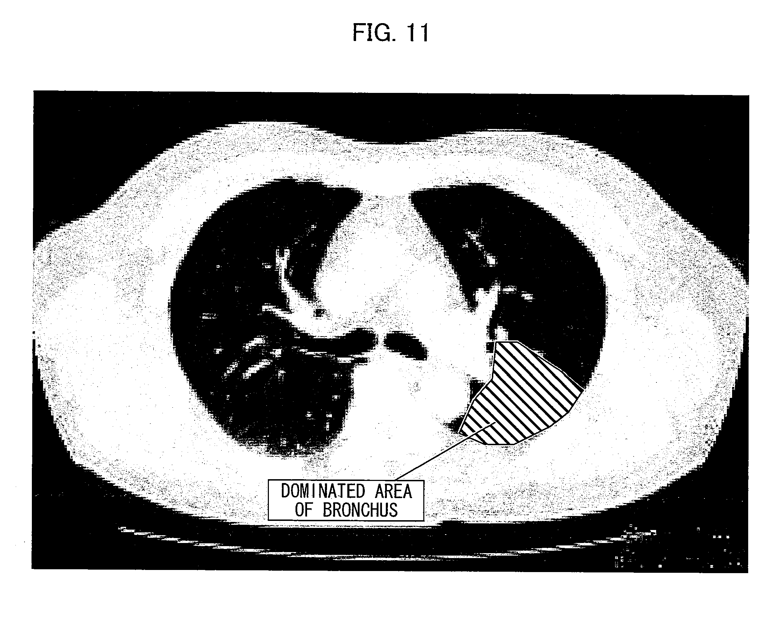

Fig. 11 is a diagram showing a display image in which the above-described dominated area of the bronchus corresponding to the position (area) of the pathological examination is displayed in a manner that enables to distinguish the bronchus from the other regions. The dominated area of the bronchus can be displayed in a manner that enables to distinguish the bronchus from the other regions, for example, by changing the density of the area or by adding a color to the area.

Note that, in the present embodiment, the distal end position of the bronchoscope is specified by an anatomical name or a symbol of a bronchial branch; however, the method for specifying the distal end position of the bronchoscope is not limited to this. The position (area) of the pathological examination can also be specified by an anatomical name or a symbol of a lung segment indicating the dominated area of the bronchus. Further, the position (area) of the pathological examination can be specified by an anatomical name or a symbol which enables to uniquely specify other pathological examination sites as well as the lung field.

### <Others>

In the above-described embodiments, when an image diagnosis, and the like, is performed with a human body image displayed, if a pathological examination has been performed in association with the displayed human body image, the information on the pathological examination is displayed. Further, when the position of the pathological examination (biopsy site) is included in the displayed human body image, the position of the pathological examination is visibly displayed by a marker, and the like. However, the presently disclosed subject matter is not limited to this. When the information on the pathological examination is displayed, the human body image corresponding to the pathological examination can be simultaneously displayed.

For example, when retrieval information, such as an examination ID and a patient ID, is inputted, then information on a desired pathological examination can be retrieved from the pathology DB 46, or when a list of information on the pathological examination registered in the pathology DB 46 is displayed by the monitor device 28, then information on the desired pathological examination can be selected from the list by using a pointing device, such as the mouse 32. When the information on the desired pathological examination is selected in this way, the information on the corresponding pathological examination is read out from the pathology DB 46, and displayed by the monitor device 28. Also, the human body image (on which the position of the pathological examination can be visibly recognized by a marker, and the like) corresponding to the pathological examination is displayed by the monitor device 28.

Further, in the present embodiment, when the position of the pathological examination (biopsy site) is included in the human body image, the position of the pathological examination is visibly displayed by a marker, and the like. However, a mode selecting device for selecting one of a display mode and a non-display mode of the position of the pathological examination can be provided, and only when the display mode is selected by the mode selecting device, the position of the pathological examination can be visibly displayed on the human body image. Thereby, when the display mode and the non-display mode are suitably switched, the position of the pathological examination on the human body image can be excellently grasped by the display mode, and the excellent image diagnosis can be performed by the image in which an obstacle, such as the marker, is eliminated by the non-display mode.

Further, in the present embodiment, the position of the pathological examination is managed (stored) on the side of the pathology DB 46, but the presently disclosed subject matter is not limited to this. Alternatively, the position of the pathological examination can be managed (stored) on the side of the image DB 44. In this case, the management item of the pathology position may be provided in the image DB 44, or the position of the pathological examination may be recorded in the tag (private tag of the DICOM file), and the like, of the image file of the human body image.

Further, in the radiographic image display apparatus 10 according to the present embodiment, the human body image, the information on the pathological examination, and the like, are acquired from the external image DB 44 and the external pathology DB 46 via the network 50; however, the presently disclosed subject matter is not limited to this. Alternatively, when the human body image, and the like, is stored in an internal storage device (the hard disk device 20), the human body image, and the like, can be acquired from the internal storage device.

Further, the presently disclosed subject matter is not limited to the above described examples. It goes without saying that various modification and changes are possible within the scope and spirit of the invention.

For example, a computer program product (for example, a ROM, flexible disk, optical disk, and so on) recorded on a computer readable medium, which includes computer-executable instructions for causing a computer to execute functions performed by the radiographic image display apparatus according to any one of the embodiments, can also achieve the aim of the presently disclosed subject matter. In this case, first, the program is installed to a computer from the computer program product, and then the computer executes the program to execute functions performed by the radiographic image display apparatus according to any one of the embodiments.

## Claims

1. A radiographic image display apparatus (10), which makes a display device (28) display a medical radiographic image, comprising:
a storing device (44, 46) which stores a position of a pathological examination on the radiographic image in association with the radiographic image;
a position acquiring device (12) which, when the radiographic image is displayed, acquires, from the storing device (44, 46), the position of the pathological examination stored in association with the radiographic image; and
a display control device (12) which, on the basis of the position of the pathological examination that is stored in the storing device (44, 46) and acquired by the position acquiring device (12), makes the display device (28) visibly display the position of the pathological examination on the radiographic image displayed by the display device (28).

2. The radiographic image display apparatus (10) according to claim 1, further comprising:
an image acquiring device (12) which acquires a radiographic image including a site of a pathological examination;
a position specifying device (12) which makes the display device (28) display the acquired radiographic image and which specifies a position of a pathological examination on the displayed radiographic image; and
a registering device (12) which makes the storing device (44, 46) store, in association with the radiographic image, the position of the pathological examination specified by the position specifying device (12).

3. The radiographic image display apparatus (10) according to claim 2,
wherein the position specifying device (12) includes a pointing device (24, 26, 30, 32) which moves a pointer to a desired position on the radiographic image displayed by the display device (28), and
wherein the registering device (12) makes the storing device (44, 46) store the position on the radiographic image specified by the pointing device (24, 26, 30, 32), as the position of the pathological examination in association with the radiographic image.

4. The radiographic image display apparatus (10) according to claim 1, further comprising:
an image acquiring device (12) which acquires radiographic images before and after the pathological examination, each of the images includes a site of a pathological examination;
a position specifying device (12) which makes the display device (28) display the acquired radiographic image after the pathological examination and which specifies the position of the pathological examination on the displayed radiographic image;
a position converting device (12) which converts the position of the pathological examination specified on the radiographic image after the pathological examination to the position of the pathological examination on the radiographic image before the pathological examination; and
a registering device (12) which makes the storing device (44, 46) store, in association with the radiographic image after the pathological examination, the position of the pathological examination specified by the position specifying device,
wherein the display control device (12) makes the display device (28) visibly display, on the radiographic image before the pathological examination displayed by the display device (28), the position of the pathological examination converted by the position converting device (12).

5. The radiographic image display apparatus (10) according to claim 1, further comprising:
an image acquiring device (12) which acquires radiographic images before and after the pathological examination, each of the images includes a site of a pathological examination;
a position specifying device (12) which makes the display device (28) display the acquired radiographic image after the pathological examination and which specifies the position of the pathological examination on the displayed radiographic image;
a position converting device (12) which converts the position of the pathological examination specified on the radiographic image after the pathological examination to the position of the pathological examination on the radiographic image before the pathological examination; and
a registering device (12) which makes the storing device (44, 46) store, in association with the radiographic image before the pathological examination, the position of the pathological examination, which position is converted by the position converting device (12).

6. The radiographic image display apparatus (10) according to claim 4 or claim 5, further comprising:
instead of the position specifying device (12), a position detecting device (12) which detects, by image processing, the position of the pathological examination from the acquired radiographic image after the pathological examination.

7. The radiographic image display apparatus (10) according to claim 2, wherein the position specifying device (12) includes a device which specifies the position of the pathological examination on the basis of an input of one of a name and a symbol which indicate an anatomical site.

8. The radiographic image display apparatus (10) according to claim 2, applied to a pathological examination performed in such a manner that the distal end of a bronchoscope is inserted to reach a desired position of the bronchus, a sterile physiological saline solution is injected into a lung segment distal to the insertion position, the injected solution is sucked back, and cells, protein, and microorganisms in the sucked back saline solution are examined,
wherein the display control device (12) includes a recognizing device which, when the distal end position of the bronchoscope inserted into the bronchus, or one of a name and a symbol that indicate the anatomical site corresponding to the distal end position of the bronchoscope is inputted by the position specifying device (12), recognizes, from the radiographic image, a dominated area of the bronchus which is distal to the distal end position of the bronchoscope, and
wherein the display control device (12) makes the display device (28) visually display the recognized dominated area of the bronchus in a manner that the recognized dominated area of the bronchus can be discriminated from the other regions on the radiographic image displayed by the display device (28).

9. The radiographic image display apparatus (10) according to any one of claims 1 to 8, further comprising:
a pathology database (46) which manages information on a pathological examination of a tissue collected from a position of the pathological examination,
wherein the display control device (12) makes the display device (28) visibly display the position of the pathological examination on the radiographic image displayed by the display device (28) and reads the information of the corresponding pathological examination from the pathology database (46) to make the display device (28) display the read information.

10. The radiographic image display apparatus (10) according to claim 9, wherein the pathology database (46) also serves as a storing device (44, 46) which stores, in association with the radiographic image, the position of the pathological examination on the radiographic image.

11. The radiographic image display apparatus (10) according to any one of claims 1 to 10, further comprising:
a pathology database (46) which manages information on a pathological examination of a tissue collected from a position of the pathological examination; and
a selecting device (12) which selects information on a desired pathological examination from the information on the pathological examination registered in the pathology database (46),
wherein when the information on the desired pathological examination is selected by the selecting device (12), the display control device (12) reads out corresponding information on the pathological examination from the pathology database (46), and makes the display device (28) display the read information on the pathological examination and the radiographic image corresponding to the information on the pathological examination.

12. The radiographic image display apparatus (10) according to claim 11, wherein the selecting device includes at least one of:
a first selecting device (12) which retrieves information on a desired pathological examination from the pathology database (46) by an input of retrieval information; and
a second selecting device (12) which includes a device which makes the display device (28) display a list of information on the pathological examination registered in the pathology database, and a device which selects information on a desired pathological examination from the list.

13. The radiographic image display apparatus (10) according to any one of claims 1 to 12, further comprising
an image database (44) which manages medical radiographic images,
wherein the position of the pathological examination on the radiographic image is recorded in a tag of an image file of the radiographic image.

14. The radiographic image display apparatus (10) according to any one of claims 1 to 13, further comprising
a mode selecting device (12) which selects one of a display mode and a non-display mode of a position of a pathological examination,
wherein the display control device (12) makes the position of the pathological examination visibly displayed on the radiographic image only when the display mode is selected by the mode selecting device (12).

15. A radiographic image display method for making a display device (28) display a medical radiographic image, comprising the steps of:
making a storing device (44, 46) store a position of a pathological examination on the radiographic image in association with the radiographic image;
acquiring, when the radiographic image is displayed, from the storing device (44, 46), the position of the pathological examination stored in association with the radiographic image; and
making the display device (28) visibly display the position of the pathological examination on the radiographic image displayed by the display device (28), on the basis of the acquired position of the pathological examination.

16. The radiographic image display method according to claim 15, further comprising the steps of:
acquiring a radiographic image including a pathological examination site;
making the display device (28) display the acquired radiographic image and specifying a position of the pathological examination on the displayed radiographic image; and
making the storing device (44, 46) store the specified position of the pathological examination in association with the radiographic image.

17. A computer program product stored on a computer readable medium for making a computer perform radiographic image display control, the product comprising computer-executable instructions of:
making a storing device (44, 46) store a position of a pathological examination on the radiographic image in association with the radiographic image;
acquiring, when the radiographic image is displayed, from the storing device (44, 46), the position of the pathological examination stored in association with the radiographic image; and
making the display device (28) visibly display the position of the pathological examination on the radiographic image displayed the display device (28), on the basis of the acquired position of the pathological examination.

18. The computer program product according to claim 17, further comprising computer-executable instructions of:
acquiring a radiographic image including a pathological examination site;
making the display device (28) display the acquired radiographic image and receiving specification of a position of the pathological examination on the displayed radiographic image; and
making the storing device (44, 46) store the specified position of the pathological examination in association with the radiographic image.
